# EUROPEAN PATENT SPECIFICATION

(11) **EP 3 161 491 B1**
(45) Date of publication and mention of the grant of the patent: **05.08.2020**
(21) Application number: 15731900.5
(22) Date of filing: 24.06.2015
(51) Int. Cl.: G01N 33/68

(54) **DIAGNOSTIC METHOD FOR THE DETECTION OF THE MANIPULATION OF MUSCLE GROWTH IN A DOMESTIC ANIMAL**
DIAGNOSEVERFAHREN FÜR DEN NACHWEIS DER MANIPULATION DES MUSKELWACHSTUMS BEI EINEM HAUSTIER
PROCÉDÉ DE DIAGNOSTIC POUR LA DÉTECTION DE LA MANIPULATION DE LA CROISSANCE MUSCULAIRE CHEZ UN ANIMAL DOMESTIQUE

(30) Priority: 24.06.2014 EP 14173757
(43) Date of publication of application: 03.05.2017
(73) Proprietor: Stichting Kwaliteitsgarantie Vleeskalversector, 3706 AR Zeist (NL)
(72) Inventor: VAN DER WEG, Cornelis Peter Van, NL-3706 AR Zeist (NL)
(74) Representative: Algemeen Octrooi- en Merkenbureau B.V.
(86) International application number: PCT/EP2015/064242
(87) International publication number: WO 2015/197683

(56) References cited:
- WO-A1-99/02667
- WO-A1-99/42573
- WO-A2-01/05820
- WO-A2-2007/044411
- US-A- 6 103 466
- TSUCHIDA KUNIHIRO: "Targeting myostatin for therapies against muscle-wasting disorders", CURRENT OPINION IN DRUG DISCOVERY AND DEVELOPMENT, CURRENT DRUGS, LONDON, GB, vol. 11, no. 4, 1 July 2008 (2008-07-01), pages 487-494, XP008120304, ISSN: 1367-6733
- DAESELEIRE E A I ET AL: "MULTIRESIDUE ANALYSIS OF ANABOLIC AGENTS IN MUSCLE TISSUES AND URINES OF CATTLE BY GC-MS", JOURNAL OF CHROMATOGRAPHIC SCI, OXFORD UNIVERSITY PRESS, CARY, NC, USA, vol. 30, no. 10, 1 October 1992 (1992-10-01), pages 409-414, XP009034224, ISSN: 0021-9665
- E. DAESELEIRE ET AL: "Validation of multi-residue methods for the detection of anabolic steroids by GC-MS in muscle tissues and urine samples from cattle+", THE ANALYST, vol. 123, no. 12, 1 December 1998 (1998-12-01), pages 2595-2598, XP055510286, ISSN: 0003-2654, DOI: 10.1039/a805164j

## Description

The invention relates to a diagnostic method and kit for the identification of the manipulation of muscle mass in a domestic animal.

Maintenance of muscle mass in domestic animals is regulated by the gene product known as myostatin (also designated as GDF-8, Lee SJ, Mc Pherron AC. Regulation of myostatin activity and Muscle Growth, Proceedings of the National Academy of Sciences and the United States of America, 2001, 98, 9306-9311).

Myostatin is one of more than 35 members of the TGF-β superfamily of growth and differentiation factors (GDF). Myostatin is part of a complex pathway of interrelated proteins that control muscle growth and degradation in animals.

If myostatin is prevented from binding to its receptor, the myostatin pathway is not activated and muscle degredation does not occur via this pathway. In animals in which the myostatin gene is inactivated, muscle growth is actually increased (Clop A, Marcq F, Takeda H, Pirottin D, Tordoir X, Bibe B, Bouix J, Caiment F, Elsen JM, Eychenne F, Larzul C, et al. A mutation creating a potential illegitimate microRNA target site in the myostatin gene affects muscularity in sheep, Nat Genet., 2006, 38, 7, 813-818).

Similarly, in animals wherein the myostatin is blocked from binding to its receptor by anti-myostatin antibodies, an increase in muscle growth is observed (Bogdanovich S, Krag TO, Barton ER, Morris LD, Whittemore LA, Ahima RS, et al. Functional improvement of dystrophic muscle by myostatin blockade, Nature, 2002, 420, 418-421).

Myostatin may be blocked from binding to its receptor by prolactin or growth and differentiation factor-associated serum protein-1 (Rodino-Klapac LR, Haidet AM, Kota J, Handy C, Kaspar BK, Mendell JR. "Inhibition of myostatin with emphasis on follistatin as a therapy for muscle disease" Muscle Nerve., 2009, 39, 3, 283-96.). In such cases where the myostatin gene is inactivated or myostatin is blocked from binding its receptor, the above pathways are not activated, resulting in muscle growth instead of muscle wasting.

As myostatin has an impact on muscle formation in animals, it has been observed that non-approved practices are used for enhancing muscle growth in animals, in particular for meat production involving immunisation of young animals, by targeting myostatin and the myostatin pathway.

A method for controlling and increasing muscle growth, especially in farm animals for slaughter, by manipulation of the myostatin pathway is taught by WO 01/05820.

WO 01/05820 provides a recombinant therapeutic vaccine comprising analogues of myostatin capable of effecting the down regulation of growth differentiation factor (GDF-8, myostatin) by stimulating production of anti-myostatin antibodies, as discussed above. The vaccines are tested for their ability to induce the production of antibodies against myostatin to identify the optimal dosing and immunization regimen for enhancing muscle growth.

As the knowledge regarding the function of myostatin and other proteins and factors involved in the myostatin pathway is applied to achieve unnaturally fast achievement of the desired muscle weight for slaughter animals, a method is needed in the art to reliably distinguish the presence of the above described abuse of the myostatin pathway so that breaches of regulatory standards can be identified.

The objective technical problem is to provide a simple test that can be conducted on an animal, for example a farm animal raised for meat production, which test will provide an indication that the muscle growth of the said animal has been artificially increased.

The inventors have found a solution to this problem by providing a diagnostic method for the detection of the manipulation of muscle growth in a domestic animal, comprising the steps of:
a) providing a tissue or bodily fluid sample from a domestic animal,
b) quantifying the amount of anti-myostatin antibodies in the sample of step a),
c) comparing the amount of the anti-myostatin antibodies measured in step b) to the amount of anti-myostatin antibodies present in at least one non-manipulated reference animal, wherein an increased amount of the anti-myostatin antibodies present in the sample relative to the non-manipulated reference animal is indicative for the manipulation of muscle growth in a domestic animal,
   wherein the method further comprises the steps of:
d) quantifying the amount of at least one additional myostatin pathway biomarker in the said sample, the said additional myostatin pathway biomarker being selected from the group consisting of myostatin propeptide, leptin, activin receptor IIB, members of the SMAD family of proteins, Alk type 1 co-receptor, Pax 3, MYOD1, MYOG, CDKN1A, CDK2 Myf-5, p-AKT, FOXO, MuRF1, FBXO32 and combinations of two or more thereof,
e) comparing the amount of the at least one additional myostatin pathway biomarker measured in step d) to the amount of the said at least one additional myostatin pathway biomarker present in at least one non-manipulated reference animal, wherein an altered amount of the said additional myostatin pathway biomarker present in the sample relative to the at least one non-manipulated reference animal is indicative for the manipulation of muscle growth in a domestic animal.

An advantage of the present method is that the manipulation of muscle growth in a domestic animal can be identified even when the history of the domestic animal being tested is not known. This is made possible by the comparison to data obtained from reference animals. Consequently the detection of the manipulation of muscle growth can be determined irrespective of the procedure that has been used to artificially enhance the muscle growth.

Procedures that may have been used to increase the amount of anti-myostatin antibodies include the immunization to stimulate the animal to produce anti-myostatin antibodies (a vaccination), or direct administration of the anti-myostatin antibodies to the animal. The anti-myostatin antibodies may prevent myostatin from being cleaved to release the active portion of the protein, or to prevent myostatin from binding to its receptor. Alternatively, the antibodies may remove secreted myostatin from circulation before it reaches the active site.

Known procedures in the prior art for determining whether a vaccination has the desired outcome require simultaneously knowing which animals have been vaccinated and which animals have not been vaccinated. The inventors have found that, using the inventive method, sampling a group of animals which potentially contains animals that have been subjected to procedures to manipulate muscle growth (for example by vaccination), and animals that have not undergone such procedures, the manipulation of muscle growth can be determined even when it is not known which animal has been treated, or what type of treatment (for example vaccination) was used. This is made possible by the comparison of the level of anti-myostatin antibodies in the animals being tested with reference levels of anti-myostatin antibodies in animals corresponding to those being tested. A significant increase in the amount of anti-myostatin antibodies above the amount found in "normal" (i.e. reference) domestic animals is indicative for the manipulation of muscle growth.

In cases where immunization has occurred with myostatin protein, a fragment of myostatin protein or a myostatin analogon, the effect of the immunization (increase in anti-myostatin antibodies) can be detected by the inventive method long even after the in vivo degradation of the myostatin immunogen.

The inventors propose that given myostatin is a native protein, normal (non-manipulated) domestic animals do not normally mount an immune response to myostatin. The endogenous concentration of anti-myostatin antibodies in non-manipulated domestic animals is therefore very low. The amount of anti-myostatin antibodies therefore functions as a biomarker of the activation or modification of the myostatin pathway. By quantifying the amount of myostatin antibodies present in a sample from an animal and comparing this amount to the amount of anti-myostatin antibodies in a reference animal, deviations in the amount of anti-myostatin antibodies can be reliably and accurately determined.

The term "manipulation of muscle growth" means a procedure to artificially enhance, by exogenous means, the natural muscle growth process in a domestic animal. Such a procedure can involve the immunization or vaccination using a myostatin immunogen.

A "myostatin immunogen" includes anti-myostatin antibodies which can be directly administered to an animal, and molecules derived from a native myostatin sequence, as well as recombinant produced or chemically synthesised myostatin polypeptides, including, for example myostatin protein and fragments thereof, analogues of myostatin protein and fragments thereof, which are able to stimulate the production of anti-myostatin antibodies in the domestic animal.

"Muscle growth" is intended to capture an increase in muscle mass relative to the reference animal. Muscles which can be quantified by carcass measurements include loin muscle area or longissimus muscle area, as determined by planimetry at the 10^{th} rib.

In step a) of the inventive method a tissue or bodily fluid sample from a domestic animal is provided.

The term "domestic animal" means an animal species that is domesticated, for example *Ovis aries* (sheep), *Sus scrofa domestica* (pig), *Capra aegagrus hircus* (goat), *Bos primigenius taurus* (cattle), *Gallus gallus domesticus* (chicken), *Equus ferus caballus* (horse), *Mus. musculus* (mouse); for example semi-domesticated *Dama dama* (fallow deer), *Cervus elaphus* (red deer), *Sus scrofa* (wild boar), *Meleagris gallopavo* (wild turkey), *Struthio camelus* (ostrich); for example undomesticated but captive-bred on a commercial scale, for example *Crocodylus porosus* (crocodile). Furthermore, "domestic animal" does not just relate to one single animal but denotes a population consisting of a specific domestic animal.

Preferably, the domestic animal is an animal raised for meat production. In a preferred embodiment, the domestic animal is an animal selected from the group consisting of cattle, sheep, horse, goat, pigs, poultry, lama, ostrich and fish, preferably cattle. Cattle includes various types of cow, and mixed breeds, including well known breeds such as Friesian, Holstein, Limousin, Fries Hollands, Holstein Friesian, Roodbont, MRIJ (Maas Rijn en Ijssel), Blaarkop, Blonde d'Aquitaine, Piemontese, and cross breeds thereof.

In an embodiment, the domestic animal is a pig, preferably of the species *Sus scrofa domestica* (domestic pig). The domestic pig also includes breeds such as Large White, Landrace, Yorkshire, Duroc, Piétrain, Gloucestershire Old Spots and cross breeds thereof.

In an embodiment, the domestic animal is a sheep, preferably breeds such as Merino, Dorper, Rambouillet, Icelandic, Ile-de-France, Gromark, Zwartbles and cross breeds thereof.

The type of tissue provided may be a hard type, for example hair, or soft type for example, muscle. Alternatively, bodily fluids, more preferably liquids, such as saliva, sweat, urine, whole blood, blood serum or blood plasma and cerebrospinal fluid may be used.

The method has the advantage that easy to obtain bodily fluid samples such as blood and urine may be used. The use of such samples is advantageous as they can be transported and stored easily so that the diagnostic method can be conducted by a laboratory remote from the location of the domestic animals. Muscle can be obtained by standard biopsy procedures. Tissue and bodily fluid samples are also amenable to automated handling making the inventive method suitable for high-throughput-screening techniques.

Furthermore, the sample can be taken from an animal for which no history is known. In other words, the diagnostic method according to the present invention is particularly useful for identifying the manipulation of muscle growth when it is not known whether an animal has been subjected to a particular treatment or not.

The quantification of the amount of anti-myostatin antibodies present in the sample conducted in step b) is not limited to a specific technique. Suitable techniques known to the skilled person include for example ELISA techniques, immunoPCR and RT-PCR for mRNA.

The term "non-manipulated reference animal" means a domestic animal that has not been subject to a procedure to artificially enhance muscle growth whereby the reference animal is of the same species as the said domestic animal. The term "reference animal" as used in the description is synonymous with "non-manipulated reference animal." Preferably, the reference animal is of same sub-species, as the animal being tested. Even more preferably, the reference animal is of the same breed as the animal being tested because the immune response to myostatin immunogen may vary across certain breeds. Also the reference animals are preferably in a comparable stage of growth development and more preferably of the same age. It is preferred that the reference animals are from the same geographical area, and have a comparable diet to the animal from which a sample was taken. It is even more preferred that the reference animal is of the same gender as the animal being tested as myostatin levels may vary between the sexes. By careful consideration of these factors such as breed, gender and age, natural variation in anti-myostatin antibody levels are taken into account, increasing the reliability of the present method.

Following the quantification of anti-myostatin antibodies present in the sample it can be determined whether the amount of anti-myostatin antibodies measured is indicative for the manipulation of muscle mass when compared to the non-manipulated reference animal data. When an increased amount of anti-myostatin antibodies is present, relative to the reference animal data, this is indicative for the manipulation of muscle growth in a domestic animal. An animal having an increased amount of anti-myostatin antibodies will be classed as "suspicious" for muscle growth manipulation. When the amount of anti-myostatin antibodies is not increased relative to reference animal data, i.e. stays the same or is decreased, the sample is classed as "negative" for muscle growth manipulation.

The reference amount of anti-myostatin antibodies is also designated as the "threshold level" and preferably comprises data from more than one reference animal. The "threshold level" can be determined as follows: the amount of anti-myostatin antibodies is determined in one or more reference animals, preferably at least three, more preferably at least five, even more preferably at least 10 reference animals, which have not been subjected to any muscle growth manipulation, over a period of at least 2 weeks, preferably 3 weeks and more preferably over 4 weeks. The reference animals are of the same species, and preferably the same gender, as the animal to be tested, as discussed above. By monitoring the reference animals over time for more than 2 weeks, possible fluctuations in the levels anti-myostatin antibodies are taken into account increasing the reliability of the reference data.

The threshold level may also be determined by data obtained by data mining techniques. For example, the amount of anti-myostatin antibodies present in a reference animal can be taken from previously taken measurements which have been stored in a database for the animals of the same species as the animals being tested.

Subsequently, the mean and standard deviation of the measured amounts of anti-myostatin antibodies (or values derived from databases) are calculated. It is advantageous to define two thresholds based on the mean ± standard deviation and the mean ± 3 x standard deviation as this enables samples to not only be classified as "suspicious" but can be defined further as being "conclusively positive". For instance, above a lower threshold defined by mean ± standard deviation, the animal can be defined as "suspicious" for muscle growth manipulation. Above an upper threshold level defined by mean ± 3 x standard deviation, the animal is determined to be "conclusively positive." Below the lower threshold (mean ± standard deviation), the animal is believed to be "conclusively negative".

The ability to determine whether a sample is "conclusively positive" or "conclusively negative" is also dependent on the statistical certainty, in other words, significance, of the measurements.

Significance may be determined using standard statistical techniques. When multiple measurements are carried out on a sample, amounts of antibodies can be presented as mean ± standard deviation (S.D). Methods such as General Linear Model (GLM) procedure of SPSS 13.0 (SPSS Inc., Chicago, IL), and the Least-Square-Differences may be used for comparison of multiple measurements. A Student's t-test can be used to evaluate the differences between the samples and reference levels. Reliable conclusive amounts of anti-myostatin antibodies may for instance be set at 97% or 99% statistical certainty. The level of statistical significance is preferably set at P < 0.10, more preferably P < 0.05.

Preferably, the statistical significance is determined by the standard deviation. The amount of anti-myostatin antibodies present in a reference sample is determined, and the standard deviation is determined. A difference of three times the standard deviation between the reference animal and the animal suspected to have been subject to muscle growth manipulation, is taken to mean that the sample is "positive" for muscle growth manipulation and that this is a statistically reliable result.

When an increase of between one but less than three standard deviations is observed, the animal is classed as "suspicious" for the presence of muscle growth manipulation.

When the difference between the amount of anti-myostatin antibodies measured in the reference animal and the animal being tested is less than one standard deviation, the sample is classed as negative for the presence of muscle growth manipulation.

For example, the amount of endogenous anti-myostatin antibodies in, 19 week old Holstein-Friesian Calves (serving as the reference animal) is typically lower than 237 ng/mL. This has been determined by analysing the anti-myostatin antibody amount in 21, 8 week old Holstein-Friesian Calves that had not been subjected to any muscle growth manipulation. These calves where monitored daily and the amount of anti-myostatin antibodies measured for a period of 19 weeks. The lower threshold value for Holstein-Friesian Calves aged 20 weeks or less was determined to be 237 ng/mL. The upper level threshold level was determined to be 385 ng/mL.

Quantities of anti-myostatin antibodies above 237 ng/mL, but less than 385 ng/mL, measured in a sample of Holstein-Friesen calves which are suspected to have been subject to exogenous muscle growth manipulation, are therefore indicative for elevated levels of myostatin, and therewith for manipulation of muscle growth. Above 385 ng/mL, samples are classed as conclusively positive. Samples below 237 ng/mL are classed as conclusively negative.

It should be noted that the diagnostic method according to the present invention is not for curative purposes. The inventive diagnostic method is carried out to identify whether breaches of regulatory standards regarding animal husbandry have occurred.

The term "anti-myostatin antibodies" means an antibody capable of specifically binding myostatin protein. Such an antibody includes polyclonal and monoclonal antibody preparations, monospecific antisera, as well as preparations including hybrid antibodies, altered antibodies, F(ab') 2 fragments, F(ab) fragments, Fv fragments, single domain antibodies, chimeric antibodies, and functional fragments thereof, which retain specificity for myostatin.

In an embodiment, the anti-myostatin antibodies quantified in step b) are chosen from the group consisting of IgA, IgD, IgE, IgG or IgM and IgG anti-myostatin antibodies, preferably IgG anti-myostatin antibodies.

The present invention is not limited to a specific type of anti-myostatin antibody. The IgG type of anti-myostatin antibody is considered to be the most preferable type of antibody to be analysed in blood serum due to the propensity for domestic animals to produce IgG type antibodies.

In cases where the amount of anti-myostatin-binding antibodies is above the reference amount, but not by a statistically significant amount, e.g. not more than three standard deviations, the animal may be diagnosed as "suspicious", as discussed above. An animal diagnosed as "suspicious" may be retested using the same diagnostic method. A retest may rule out mistakes that may occur in sample taking or measurements. Another means to further increase the reliability the present diagnostic method is to determine the knock on effects of an increase of anti-myostatin antibodies on proteins in the myostatin pathway.

To briefly summarize the myostatin pathway, myostatin binds to the activin type IIB transmembrane receptor, which then recruits and activates the ALK type I co-receptor by phosphorylation. This results in the recruitment of phosphorylated SMAD2 and SMAD3 proteins, consequently down-regulating Pax3 and Myf-5 proteins, ultimately resulting in muscle wasting (Han H. Q, Mitch W. E, Targeting the Myostatin Signalling Pathway to Treat Muscle Wasting Diseases, Curr Opin Support Paliat Care, 2011, 5, 4, 334-341).

Other important proteins in this pathway are MyoD1 and CDK2, sustained expression of these is necessary for retaining the expression of muscle-related genes. MyoG is a muscle-specific transcription factor that can induce myogenesis in a variety of cell types in tissue culture and it is essential for the development of functional skeletal muscle. A further relevant protein of the myostatin pathway is CDKN1A (also known as p21). Myostatin is negatively regulated by myostatin propeptide and can also be blocked by leptin.

A second consequence of myostatin binding to its receptor is that p-AKT activity decreases, which consequently reduces FOXO phosphorylation. De-phosphorylated FOXO enters the nucleus to activate transcription of atrophy-specific E3 ligases MuRF1 and FBXO32, which cause muscle protein ubiquitination and thus subsequent degradation of muscle. p-AKT, FOXO, MuRF1 and FBXO32 are further biomarkers of the myostatin pathway.

By measuring the amount of anti-myostatin antibodies and the amount of additional biomarkers of the myostatin pathway, the reliability of the present diagnostic method is further increased, in particular useful when the diagnosis based on the antibody alone would be suspicious and not conclusive.

The inventors have found that following the quantification of anti-myostatin antibodies in a sample taken from a domestic animal, it is advantageous to determine the amount of at least one additional myostatin pathway biomarker present in the sample. In this way, the result from step c) can be confirmed and an even more reliable result obtained in suspicious cases.

The term "additional myostatin biomarker" means a mRNA molecule, peptide, polypeptide or protein that is involved in the same biochemical pathway as myostatin. If the additional myostatin biomarker is a protein, the protein may be phosphorylated or dephosphorylated. The change in one or more of these biomarkers, or its degree of phosphorylation, relative to a reference animal, is therefore indicative for the manipulation of muscle growth.

A further advantage of this combination method whereby first the amount of anti-myostatin antibodies is measured followed by the amount of additional myostatin biomarkers, is that the observed changes in the measured biomarkers can be distinguished from pathological / physiological responses in a domestic animal thus further improving the reliability of the method so that it can be stated with confidence that a domestic animal has been subjected to muscle growth manipulation.

The additional myostatin biomarker is chosen from the group of myostatin propeptide, leptin, activin receptor IIB, members of the SMAD family of proteins, Alk type 1 co-receptor, Pax 3, MYOD1, MYOG, CDKN1A, CDK2 Myf-5, p-AKT, FOXO, MuRF1, FBXO32 and combinations of two or more thereof. Preferably the SMAD family proteins are SMAD2, SMAD3. In the context of the present application, the SMAD family of proteins also includes phosphorylated SMAD proteins, preferably phosphorylated SMAD2 and phosphorylated SMAD3.

The additional myostatin biomarkers can be measured by using techniques known to the skilled person, for example ELISA assays, Western blot, Northern Blot or Southern Blot, PCR directed towards detecting the biomarkers at the protein or gene transcription level or transcriptomics methods using micro-arrays or RNA-seq.

By "an altered amount of the said additional myostatin pathway biomarker" is meant the relative increase or decrease in the said additional myostatin pathway biomarkers in an animal being tested, as compared to a reference animal.

The relative increase or decrease may be different for different species, and/or breeds. It has been found that for some additional myostatin pathway biomarkers, the amount will be lowered as compared to a reference animal when the myostatin pathway is manipulated in order to achieve muscle mass increase, such biomarkers being myostatin propeptide, phosphorylated SMAD2 and 3, alk type 1 co-receptor, MuRF1 and FBXO32.

The inventors have further found that the amount of certain myostatin pathway biomarkers increases with respect to a reference animal when the myostatin pathway is manipulated, such biomarkers being, leptin, p-AKT, phosphorylated FOXO, MYOD1, CDKN1A, MYOG, CDK2, Pax 3 , Myf-5, activin IIB receptor.

The inventors have found that by comparing the amount of an additional myostatin pathway biomarker to the amount of the corresponding additional myostatin pathway biomarker in at least one non-manipulated reference animal, the manipulation of muscle growth can be further determined. Testing for multiple biomarkers further improves the reliability of the diagnostic method. Statistical methods familiar to the person skilled in the art can be applied to analyse the amounts measured, as discussed above.

In an embodiment, step d) of the diagnostic method comprises at least two myostatin pathway biomarkers and step e) comprises comparing the amount of the at least two myostatin pathway biomarkers measured in step d) to the amount of the said at least two additional myostatin pathway biomarkers present in at least one non-manipulated reference animal, respectively.

The inventors have found that by comparing at least two additional myostatin pathway biomarkers with the amounts for these biomarkers in a reference animal the reliability of the test is further increased. Particularly preferred biomarkers are selected from the group consisting of myostatin propeptide, activin IIB receptor, leptin, phosphorylated SMAD2 and SMAD3, MuRF1 and FBXO32.

In an embodiment, the two additional myostatin pathway biomarkers are SMAD2 and SMAD3 proteins. When a decrease in phosphorylated SMAD2 and SMAD3 protein is measured in a sample, preferably a tissue sample, from a domestic animal compared to a reference animal, this is indicative for the presence of manipulation of muscle growth.

In an embodiment, the two additional myostatin pathway biomarkers are leptin and activin IIB receptor. When an increase in leptin and activin IIB receptor is measured in a sample from a domestic animal compared to a reference animal, this is indicative for the presence of the manipulation of muscle growth.

In an embodiment, at least three additional myostatin pathway biomarkers, preferably at least four additional myostatin pathway biomarkers, more preferably five additional myostatin pathway biomarkers, even more preferably six additional myostatin pathway biomarkers and most preferably seven additional myostatin pathway biomarkers are quantified in step d).

The reliability of the method according to the present invention can be further improved by, quantifying at least three additional myostatin pathway biomarkers, for example myostatin propeptide, activin type IIB receptor and leptin, a reliable indication of muscle growth manipulation can be obtained.

A yet more reliable result and be achieved when preferably four, more preferably five, even more preferably six, yet more preferably seven additional myostatin pathway biomarkers are used. For example, when myostatin propeptide, activin receptor IIB leptin, phosphorylated SMAD2 & phosphorylated SMAD3, Pax 3, MyoD1 and MuRF1 are measured, a particularly accurate profile of the myostatin pathway biomarkers is obtained so that the presence of muscle growth manipulation can be determined with a high degree of certainty. Particularly reliable results are achieved using eight, preferably nine, more preferably ten, even more preferably eleven, yet more preferably twelve, even yet more preferably thirteen, still more preferably fourteen and most preferably fifteen additional myostatin pathway biomarkers are used. Increasing the number of biomarkers decreases the likelihood of a false positive or false negative results. For example, when myostatin propeptide, leptin, activin receptor IIB, phosphorylated SMAD2 & phosphorylated SMAD3, Alk type 1 co-receptor, Pax 3, MYOD1, MYOG, CDKN1A, CDK2 Myf-5, p-AKT, phosphorylated FOXO, MuRF1, FBXO32.

The biomarkers in the myostatin pathway are not only responsive to direct disruption of myostatin binding but may also be effected by pathological processes. The reliability of the diagnostic method is yet further improved by determining the relationship between the amount of anti-myostatin antibodies and the amount of the other biomarkers in the myostatin pathway. In this way it is possible to carry out a test for the manipulation of muscle growth in an animal that is independent of pathological processes.

In an embodiment, the diagnostic method further comprises the step f) of correlating the amount of anti-myostatin antibodies quantified in step b) with the amount of one or more additional myostatin pathway biomarkers quantified in step d) therewith providing a profile of the relationship between the relative amounts of the said anti-myostatin antibodies and the said one or more additional myostatin pathway biomarkers, and g) comparing the profile in step f) to a reference profile obtained by correlating the amount of said anti-myostatin antibodies with the amount said of one or more additional myostatin pathway biomarkers quantified in at least one non-manipulated reference animal,
wherein the manipulation of muscle growth in a domestic animal can be determined when the profile of step f) differs from that of step g).

The inventors have further found that the relationship between the amount of anti-myostatin antibodies and the additional myostatin pathway biomarkers, in particular phosphorylated SMAD2 and SMAD3, are also characteristic of a procedure to artificially enhance muscle growth in a domestic animal.

It has been found that when the profile of step f) differs from that of step g), in other words, the relationship between amounts of the anti-myostatin antibodies and the additional myostatin pathway biomarkers is altered in the sample being tested, relative to the reference, this is indicative for muscle growth manipulation. In this way, the presence of muscle growth manipulation can be determined by using the amount of anti-myostatin antibodies, the amount of additional myostatin pathway biomarkers, and reference data for these amounts in non-manipulated animals, without the need for a "positive" control, animals that are known to have been manipulated. This is advantageous as the method according to the invention can detect muscle growth manipulation that has been triggered by different means, for example vaccination with myostatin or direct injection of anti-myostatin antibodies.

In a sample where the amount of anti-myostatin antibodies is increased relative to the reference, and the amount of additional biomarkers also varies according to the reference, for example myostatin propeptide decreases, activin IIB receptor and leptin increases, it can be said that such a profile is characteristic for muscle growth manipulation via the myostatin pathway.

The inventors have found that when only the amount of additional myostatin pathway biomarkers, e.g. myostatin propeptide, activin IIB receptor and leptin, are different to the reference data, but there is no increase in the amount of anti-myostatin antibodies, it can be concluded that there has been no manipulation of muscle growth. In this way, it is possible to distinguish between pathological fluctuations in myostatin pathway biomarkers and manipulation of the myostatin pathway using exogenous methods.

In another embodiment, the ratio of anti-myostatin antibodies to additional biomarkers, for example, phosphorylated SMAD2 can be determined. The ratio obtained can be compared to the ratio of myostatin antibodies to phosphorylated SMAD2 protein in a reference animal. The difference between the two ratios, in addition to the level of anti-myostatin antibodies, provides an additional indication for the manipulation of muscle growth.

This comparison can be repeated for multiple biomarkers so that a profile is built up to map the relative changes in the ratios in the sample compared to the reference data.

Each sample is subjected to preferably at least two measurements to increase the reliability of the method. The data generated can be subjected to standard statistical techniques that are known to the skilled person, as discussed above.

In an embodiment, step d) comprises at least two additional myostatin pathway biomarkers and step f) comprises correlating the amount of the at least two myostatin pathway biomarkers measured in step d) with each other therewith providing a profile of the relationship between the relative amounts of the said at least two additional myostatin pathway biomarkers.

It has been found that determining the relationship between the amounts of the additional myostatin pathway biomarkers in the sample provides a yet improved reliable means of identifying domestic animals which have been subject to a procedure to manipulate muscle growth.

The relative changes with respect to each of the additional myostatin biomarkers is also indicative for muscle growth. For example, following the determination of the relationship between the amount of anti-myostatin antibodies and additional myostatin pathway biomarkers, the relationship *between* the respective additional myostatin pathway biomarkers can be determined. For instance, when the amount of myostatin propeptide decreases with respect to activin IIB receptor and leptin, it can be concluded that muscle growth manipulation via the myostatin pathway has occurred. In an embodiment, the amount of phosphorylated SMAD2 and SMAD3 proteins are compared to the amount of prolactin protein. It has been found that by comparing the amounts of these biomarkers measured in a sample, to the amount present in a reference animal, the presence of the manipulation of muscle growth can be determined.

In a generic example the amount of anti-myostatin antibodies in sample may be defined by the value α^{s}. The amount of the additional myostatin pathway biomarker in a sample may be defined by the value β^{s}ₙ, where n is a specific additional myostatin pathway biomarker. The ratio α^{s} : β^{s}ₙ defines the relationship of the anti-myostatin antibodies in sample to the amount of a specific additional myostatin pathway biomarker. In a case where two additional myostatin pathway biomarkers are quantified, the ratios are defined as α^{s} : β^{s}₁ and α^{s} : β^{s}₂..

The reference profile may be generated from data from a reference animal, to define the corresponding ratio for the reference animal to the corresponding ratio α^{r} : β^{r}ₙ. Using the illustrative example of two additional myostatin pathway biomarkers, the ratios are defined as α^{r} : β^{r}₁ and α^{r} : β^{r}₂.

In an embodiment, the ratio α^{r} : β^{r}ₙ may be generated by data-mining of proteomic databases which detail expression profiles for additional myostatin pathway biomarkers in myostatin (de)regulated systems.

Correlation of the profiles generated by analysis of the sample and of the reference animal can be done using standard statistical techniques that are known to the skilled person. For example, the correlation may be defined as (α^{s} : β^{s}₁) : (α^{r} : β^{r}₁), (α^{s} : β^{s}₂) : (α^{r} : β^{r}₂) etc..

The correlation may result in a numerical or graphical (plot) representation. For example, a plot of the relative difference in the observed relationship (α^{s} : β^{s}ₙ) and the reference relationship (α^{r} : β^{r}ₙ), normalized to the reference for each additional myostatin pathway biomarker generates a characteristic profile for each sample.

When statistical analysis shows that the profile generated by analysis of the sample (step f) is significantly different i.e. by factor of three standard deviations or has a statistical significance of P<0.05, to that of the reference profile, the sample can be classified as "positive". In other words, it can be determined that the animal has been subjected to artificial manipulation of muscle growth.

The profile of a number of biomarkers can be defined by measurements of samples taken from reference animals or be taken from known values (datamining), for example by extraction from an electronically accessible database. The values for the tested animals can be fed into a computer having suitable software and a profile can be generated and compared with a corresponding reference profile, or a reference profile can be determined based on data stored in the electronically accessible database.

In an embodiment, the domestic animal is an animal raised for meat production. There is increasing consumer demand for ethically sourced, traceable and quality assured meat. The present inventive method meets this need.

In an embodiment, the domestic animal is an animal selected from the group consisting of cattle, sheep, horse, goat, pigs, poultry, lama, ostrich and fish, preferably cattle. Given the consumer demand for meat such as beef, mutton, horse, pork, chicken and fish the method according to the present invention provides means to reassure consumers that the meat has not been produced by artificial, non-approved methods in the raising of cattle, sheep, horse, goat, pigs, poultry, lama, ostrich and fish.

In an embodiment, the domestic animal is a young animal, preferably younger than one year old. As young animals are growing fast, the effect on muscle growth has a relatively large impact when compared to adolescent or adult animals.

In an embodiment, the reference animal is of the same species as the domestic animal, preferably the same species, preferably sub-species more preferably the same breed, yet more preferably also the same gender and even more preferably also the same age. Given that the amount of anti-myostatin antibodies may vary from species to species, it is important that the reference animal is of at least the same species and preferably the same sub-species.

Furthermore, variations in the amount of anti-myostatin antibodies and/or additional myostatin pathway biomarkers may exist in domestic animals of differing breed, gender and age and it is therefore desirable that the reference animal corresponds to the sample in so far as is possible.

In an embodiment, the sample is a bodily fluid selected from the group consisting of saliva, sweat, urine, whole blood, blood serum, blood plasma and cerebrospinal fluid. Samples which are easy to obtain are particularly preferred and therefore bodily fluids are preferred over tissue samples.

In an embodiment, the sample comprises blood serum. A particularly preferred sample is blood serum as anti-myostatin IgG antibodies can be found in this type of sample.

In an embodiment, the sample is a soft tissue, preferably muscle tissue. The inventors have found that muscle tissue is a preferred sample type when the additional myostatin pathway biomarker is quantified via, for example, mRNA levels.

In an embodiment, step b) comprises the steps of:
i) providing a solid surface, preferably a micro-titre plate, comprising a first binding agent capable of specifically binding the anti-myostatin antibodies to be quantified,
ii) contacting the solid surface with the sample from the domestic animal,
iii) washing the surface obtained in step ii),
iv) contacting the surface obtained in step iii) with a first detector agent capable of specifically binding the anti-myostatin antibodies,
v) quantifying the signal emitted by the first detector agent.

The inventors have found that the method according to the present invention may be carried out completely on the solid phase. Consequently, the present invention is considerably easier and quicker to carry out than a solution phase assay with detection via western blot type techniques as there is no need for the time consuming and laborious transfer to membranes.

The term "solid surface" does not imply any specific limitations, and may include any insoluble polymer material, for polyamide or a vinyl polymer (e.g., poly(meth)acrylate, polystyrene and polyvinyl alcohol, or derivatives thereof), a natural polymer such as cellulose, dextran, agarose, chitin and polyamino acids, or an inorganic polymer, such as glass or metallohydroxide.

The solid surface may be a micro-titre plate, for example a 96, 384 or 1536 well plate. Such a multiple well plate has the advantage that multiple samples can be analysed simultaneously increasing the through-put of the diagnostic method.

The sample is usually pre-treated, for instance by dilution in a suitable buffer, prior to contacting the sample with the solid surface. Suitable buffers are, for example phosphate, tris and ammonium carbonate buffers.

Washing is typically done by a buffer solution. By washing, non-binding sample matter, in particular antibodies not binding to myostatin, are removed. The determination of the antibodies is typically done by chemically coupling a detectable label to the antibodies.

In an embodiment, the first binding agent is an antibody, epitope or protein fragment capable of specifically binding the anti-myostatin antibodies. Methods to immobilize antibody, epitope or protein fragments such as myostatin propeptide on a solid surface are known in the art.

In an embodiment, the surface further comprises at least one additional binding agent capable of specifically binding the at least one additional myostatin pathway biomarker. It is particularly preferred that the solid surface, for example micro-titre plate, is also covered with an antibody, epitope or protein fragment capable of specifically binding the at least one additional myostatin pathway biomarker.

In an embodiment, step iv) comprises contacting the sample with at least one additional detector agent capable of specifically binding the at least one additional myostatin pathway biomarker. It is preferred that a specific detector reagent for the at least one additional myostatin pathway biomarker is used so that there is no cross-reactivity with the first detector agent capable of specifically binding the anti-myostatin antibodies.

In an embodiment, the first binding the agent comprises a crosslinking reagent for crosslinking with the first detector agent in the presence of the anti-myostatin antibodies. The advantage of crosslinking the first detector agent with the first binding agent is that a highly stable conjugate is formed which enhances the sensitivity of the method.

In an embodiment, the crosslinking agent is biotin. Various crosslinking agents are known to the skilled person, for example biotin or a biotin analogue. The biotin analogue preferably retains the ability to bind specifically to avidin, streptavidin or a biotin-binding fragment or mutant thereof.

In an embodiment, the at least one additional binding agent is an antibody, epitope or protein fragment capable of specifically binding the at least one additional myostatin pathway biomarker. It is preferred that the specificity of the at least one additional binding agent is such that there is no cross-reactivity between the binding agents for the respective additional myostatin pathway biomarkers.

In an embodiment, the first detector agent, and optionally the at least one additional detector agent, comprises a means to generate a quantifiable signal, said means being selected from the group consisting of polynucleotides detectable by immuno-PCR, fluorophores detectable via fluorescence spectroscopy and enzymes detectable via UV spectroscopy.

A particularly preferred technique is immunoPCR, ELISA or LC/MS/MS, for example uHPLC/MS/MS. Immuno-PCR is a very sensitive technique suitable for detecting very low concentrations of analyte making this technique particularly suitable to the inventive method.

Fluorescent labels (fluorophores) give a reliable signal that can be correlated to the concentration of the substance being measured, for example anti-myostatin antibodies or the at least one additional myostatin pathway biomarker.

The detector agent may also be coupled to an enzyme label. The enzyme may have a specificity for a particular substrate, whereby the enzyme cleaves said substrate and releases a chromophore detectable via UV spectroscopy. Such techniques are known to the skilled person, for example ELISA.

In an embodiment, said means to generate a quantifiable signal is provided by polynucleotides. Polynucleotides can be detected via immune-PCR at very low concentrations, down to for example less than 1 nanogram, or even for example 100 picograms and are therefore the preferred means to generate a quantifiable signal.

In a second aspect, the invention provides a diagnostic kit for performing a diagnostic method according to the present invention, wherein the kit comprises:
i) a solid surface comprising at least a first binding agent capable of specifically binding anti-myostatin antibodies,
ii) a holder comprising a first detector agent capable of specifically binding anti-myostatin antibodies,
iii) a set of instructions for the use of the kit containing values for the minimum amount of anti-myostatin antibodies that are required to be present in the sample for manipulation of muscle growth to be determined,
wherein the kit further comprises:
- at least one additional binding agent capable of specifically binding at least one additional myostatin pathway biomarker to be quantified and at least one additional detector agent capable of specifically binding at least one additional myostatin pathway biomarker to be quantified.

In addition the kit may comprise materials for treating samples, buffers, positive and negative control samples. The kit provides a user with the solid surface, for example micro-titre plate, and the required detector agent for detecting anti-myostatin antibodies so that the user can reliably put the inventive method into practice.

By providing the reference value of anti-myostatin antibodies, the user can easily determine whether the domestic animal from which the sample was taken had been subject to a procedure to manipulate its muscle growth.

In an embodiment, the first detector agent and the at least one additional detector agent comprises a means to generate a quantifiable signal, said means being selected from the group consisting of polynucleotides detectable by immuno-PCR, fluorophores detectable via fluorescence spectroscopy and enzymes detectable via UV spectroscopy.

In an embodiment, the kit comprises reference data for the amount of the at least one additional myostatin pathway biomarker and optionally further comprises one or more reference profiles of non-manipulated reference animals, said reference data and/or reference profiles being provided in the set of instructions or on a computer readable medium supplied with the kit. In so doing, the user of the kit is provided with the necessary reference data so that he can determine whether an animal has been subject to muscle growth manipulation or not.

The amount of the at least one additional myostatin pathway biomarker can be provided by supplying with the kit a CD, DVD, USB memory stick or other such computer readable medium. Likewise, the reference profile of the amount of anti-myostatin antibodies relative to the amount of the one or more additional myostatin pathway biomarkers in a non-manipulated reference animal, the profile of the amount of the one or more additional myostatin pathway biomarkers present in a non-manipulated reference animal, and the profile of the relationship between the relative amounts of at least two of the additional myostatin pathway biomarkers can all be provided on such computer readable media. Alternatively, the kit can comprise instructions for accessing an electronically accessible database from which the relevant reference data can easily be obtained. Further, the kit can comprise computer readable software, or instructions as how to access such software on the internet, e.g. by supplying instructions with regard to the corresponding internet address and/or supplying a password code, which software can be used to process the data obtained by the method of the invention, such as preparing an above-describe profile thereof, and comparison of the data or profile with reference data or profile.

The invention is further described by the following non-limiting examples.

### EXAMPLES

### Example 1: Quantification of anti-myostatin antibodies

In order to generate reference values for the amount of anti-myostatin antibodies present in at least one non-manipulated reference animal, calves were treated with a known amount of a myostatin blocking agent (SEQ ID No. 1 CKMSPINMLYFNGKEQIIYG - myostatin propeptide fragment). Such inhibition of the myostatin pathway leads to lower active myostatin levels, as is done by standard immunization techniques. Samples from the treated animals were compared with control animals belonging to the same breed.

### 1.1 Preparation of bovine test animals.

Calves were randomly assigned into treatment groups. Twenty one (21) Holstein-Friesian calves (12 male and 9 female) between 9 - 14 weeks of age were included in the study. 7 animals were used as a control group, whereas 14 animals were to be immunized in order to lower active myostatin levels. The control group was only injected with the injection fluids, whereas the second group was treated with the myostatin propeptide fragment. The treatments were administered intramuscularly on day 0, day 35 and day 88 of the treatment. Animals were monitored during 19 weeks from the start of the treatment.

The calves were 8 weeks old at the start of the experiment. Before and during the treatment, the calves were fed using equal and controlled amounts of commercially available CMR (Calf Milk Replacer) and Fibramix.

All calves were treated with the myostatin propeptide fragment three times with during the observation period. Physical data and samples were collected during the life-time of the calves, such as general health, local reactions, and body weight. Serum samples were collected. Some clinical signs were observed, but they remained within a range that was considered normal for a 19 weeks fattening period. The applied treatments did not impact the general health of the calves. The animals were healthy at the time of slaughter and all slaughter samples were collected.

### 1.2 Chemicals

Myostatin propeptide fragment, derived from the myostatin polypeptide sequence, having the sequence CKMSPINMLYFNGKEQIIYG (SEQ ID NO 1), was obtained from Genaxxon bioscience GmbH, Ulm, Germany, as the free acid with the N-terminus conjugated with a KLH group (keyhole limpet hemocyanin), purified by HPLC.

Myostatin propeptide fragment (SEQ ID NO 1) was dissolved in sterile arachis oil, and mixed with ViteSel, Freund's Adjuvant Incomplete, or Freund's Adjuvant Complete for injection. These materials are all commercially available. Vitesel is a commercially available vitamin injection fluid for calves containing alpha-tocoferolacetate and selenium (available from Norbrook, Inc., Kansas, USA). The control group was injected with the same injection fluid, but without the added peptide.

### 1.3 Sampling

Multiple blood samples were collected at weekly intervals and immediately stored at selected times during the treatment. At the end of the observation period at 19 weeks, all study animals were slaughtered in a licenced slaughterhouse, and tissue samples were taken.

### Preparation of tissue samples

Briefly, one aliquot (∼1 g) muscle was homogenized on ice in 20 vol of homogenizing buffer. After centrifugation (2500 g for 10 min at 4°C), the supernatant was removed and stored at at -80°C+/-5°C until further processing, according to p. 17, section 3, Methods in Molecular Biology, vol. 244: Protein Purification Protocols: Second Edition, 2004, Edited by: P. Cutler© Humana Press Inc., Totowa, NJ.

### Preparation of Serum

Blood was allowed to clot at room temperature and serum was separated by centrifugation. Each serum sample was divided into aliquots and stored at -80°C+/- 5°C until further processing.

### 1.4. Testing for anti-myostatin antibodies

The collected blood serum from the calves in section was tested for anti-myostatin antibodies.

### 1.5 Materials and Reagents

The measurements were preformed on an Imperacer® Workstation (catalog number 25-001; Supplier: Chimera Biotec GmbH, Germany), using an Imperacer® Assay Development Kit (ADK; catalog number 20-007; Supplier: Chimera Biotec GmbH, Germany) which comprises a number of buffers and is further described in EP 2 189 539, example 2. For detection, the Alternate Imperacer® Detection Conjugate "CHI-Bovine-IgG" (conjugated antibody: anti-Bovine IgG-heavy and light chain Antibody, Sheep Polyclonal, catalog number: A10-115; Supplier: Bethyl Laboratories, Inc., USA) was used. The Capture Reagent for binding anti-myostatin antibodies to the solid surface was GDF-8 (myostatin); (catalog number: 788-G8-010/CF; Supplier: R&D Systems, Germany). PCR-Mastermix (Item. No. C-022; Supplier: Chimera Biotec GmbH, Germany) The used equipment, reagents and buffers could be replaced by other commercially available materials.

### 1.6 Analytical Assay Procedure

### Step 1: Plate preparation (I)

Microplates were coated with GDF-8 (myostatin) at: 1µg/ml (30 µl/well), and incubated on the Imperacer® plate material for >12h at 4°C.

### Step 2: Plate preparation (II)

The coated microplate wells were coated with Imperacer® wash buffer "A" using washer-program "block", block against unspecific interaction with "chimera direct block" solution (240 µl/well, 1 min/RT) and with Imperacer® wash buffer "B". After this treatment, the microplate wells were ready for sample incubation.

### Step 3: Sample Incubation

Serum samples were diluted with sample dilution buffer SDB3100 in low-bind vials and incubated for 1h at room temperature in GDF-8 coated wells, 30 µl/well in double determination, using orbital shaking (750 rpm).

### Step 4:Detection Conjugate

After incubation with sample, the micro plates were washed with Imperacer® wash buffer "B". Incubate wells with Imperacer® detection conjugate "CHI-Bovine-IgG" (1/30 dilution in Conjugate Dilution Buffer) for 30min at room temperature.

### Step 5: Preparation for PCR

The wells were subsequently washed with Imperacer® wash buffers "B" and "A". 30 µl/well of ready-to-use Imperacer® PCR-mastermix were added to each well. The wells were sealed using real-time PCR foil, and placed into the Imperacer® reader 50 cycles at 95°C with 12 sec per cycle, at 50°C for 30 sec and at 72°C for 30 sec.

The samples were analysed in duplicate following the analytical assay procedure described above, including negative (no treatment with myostatin propeptide fragment) and positive controls (sample known to be treated with myostatin propeptide fragment). All samples from a single subject were be run in one assay whenever possible; multiple subjects may be run in one assay.

### 1.7 Results

For assay evaluation, a blind and an open test were performed. All measured signals were normalized by subtraction of the average negative control signal.

**Table 1 Quantification of anti-myostatin antibodies in calves**

| Sample | Amount of antibodies measured in sample / ng/ml | Classification relative to lower threshold 237 ng/ml & upper threshold 382 ng/ml |
|---|---|---|
| 1.1 (reference) | 237 | - |
| 1.2 (positive control) | 382 | Positive |
| 1.3 (unknown) | 385 | Positive (conclusive) |
| 1.4 (unknown) | 300 | Suspicious |
| 1.5 (unknown) | 225 | Negative |

The analytical assay procedure response data were recorded as Imperacer® instrument response (ΔCt; delta-Ct). The instrument response was converted into ng/ml using a standard curve. The sample status was determined using a lower analytical threshold value 237.1 ng/ml; below this threshold value the sample was judged to be negative, whereas values above this value were identified as suspicious. Samples above the upper analytical threshold value of 382.1 ng/ml were determined as conclusively positive (Sample 1.3), whereas values between the two values were deemed to be suspicious (Sample 1.4).

Both in the blind and open tests, a clear association were observed between the levels of measured anti-myostatin IgG antibodies and treatment with the synthetic peptide, compared to the negative control animals.

As can be seen in table 1 samples 1.3 and 1.4 showed a significantly higher level of anti-myostatin antibodies compared to the reference animals (sample 1.1) that had received the negative control preparation. An increase in anti-myostatin antibodies was observed during the treatment, e.g. samples taken at a later time during the study showed higher anti-myostatin IgG levels.

Depending on selection of "Threshold Level", positive animals can be identified. "Prudent" threshold levels (e.g. "progressive") result in higher numbers of false-positive controls, whereas "higher" Threshold levels ensure only true positives, but will not identify all treated animals depending on response to treatment.

Suspicious animals may need additional testing, for instance for additional biomarkers or histological markers (e.g. muscle fibre size diameter), to conclusively determine a positive or negative result. Combining various biomarkers and other parameters provides further certainty on the manipulation of muscle mass through alterations of the myostatin levels. This was done in example 3.

### Example 2 Quantification of anti-myostatin antibodies and additional myostatin pathway biomarkers

In this assay not only is the amount of anti-myostatin antibodies measured, but the amount of additional myostatin pathway biomarkers is also measured.

### 2.1 Preparation of mouse test animals

Mice were immunized with myostatin, and samples from the treated mice were subjected to multiple assays for various biomarkers related with the myostatin pathway.

### 2.2 Treatment of mice

In order to assay biomarkers related to myostatin, adult male BALB/c mice were immunized using a synthetic myostatin peptide as used above. The synthetic peptide derived from myostatin, having the sequence CKMSPINMLYFNGKEQIIYG (SEQ ID NO 1), was obtained from Genaxxon bioscience GmbH, Ulm, Germany, as the free acid with the N-terminus conjugated with a KLH group (keyhole limpet hemocyanin), purified by HPLC. For negative control, a group untreated mice were used. Adult Mice treated with siRNA were used as a positive control. The synthetic peptide was applied by a single injection at a dose of 40 µg/ protein/Mouse in Freunds complete adjuvant (FCA). Application of siRNA was done via osmotic micro pumps for 28 days. Each group contained 10 mice. After 28 days, blood serum was isolated from the mice and subjected to the biomarker test as described below.

### Reagents

Microplates and reagents were obtained from Chimera Biotec GmbH, Germany as follows: Microplate Modules (Item No. C-001), Microplate Module Frame (Item No. C-002), PCR-Foil, Storage-Foil, Buffer Reservoir (Item No. C-003), Coating Buffer (Item No. C-010); Wash-Buffer A (20x concentrate) (Item No. C-011), Wash-Buffer B (20x concentrate) (Item No. C-012); Chimera Direct Block (Item No. C-013), AnySource® Sample Dilution Buffer: SDB6000 Basic (Item No.: tbd), Ready-to-use aliquots for 16 wells (2 microplate modules) each, Conjugate Dilution Buffer-b (CDB-b) (Item No. C-021), PCR-Mastermix (Item. No. C-022), Various, analyte-specific Imperacer® Detection Conjugates (e.g. "CHI-Leptin"). Various analyte-specific capture antibodies are commercially available.

Measurements were performed on an Imperacer® Workstation (Chimera Biotec GmbH, Germany; Cat. No. 25-001 or 25-002, using various digital pipettes, multichannel pipettes, a microplate compatible PCR-thermocycler, and a multichannel microplate washer, and other standard laboratory equipment according to the method described in EP 2189539 B1 example 3. Commercially available protein low bind cups were used for minimization of unspecific binding of low antigen or reagent concentrations against the surface of the cups.

### 2.3 Assay procedure

Wash buffers were brought to room temperature before use and diluted. Wash-Buffer A was diluted by adding 50 ml of Wash-Buffer A (20x concentrate) into 950 ml deionized and DNase-free water (e.g. autoclaved) to prepare 1000 ml of Wash-Buffer A. Wash-Buffer B was diluted by adding 100 ml of Wash-Buffer B (20x concentrate) into 1900 ml deionized and DNase-free water (e.g. autoclaved) to prepare 2000 ml of Wash-Buffer B. All aliquoted components were stored on ice during the assay procedure. All samples, standards and controls were performed in duplicate.

### (I) Immobilization of Capture Antibody

The analytical Target of the Capture Antibody (Antigen) were coated from stock concentrated solutions that are commercially available. The list below shows the concentrations of the coating solutions and dilution factors for coating. The stocks were diluted with coating buffer.

**Table 2 Biomarkers analyzed**

| Biomarker | Concentration | Dilution |
|---|---|---|
| Goat anti-mouse | 5µg/ml, c = 1mg/ml | 1 : 200 |
| Leptin | 10µg/ml, c = 200µg/ml | 1 : 20 |
| IGF1 | 5µg/ml, c = 1mg/ml | 1 : 200 |
| Adiponectin | 5µg/ml, c = 1mg/ml | 1 : 200 |
| Activin II B | 5µg/ml, c = 1mg/ml | 1 : 200 |
| Myostatin Propeptide | 5µg/ml, c = 1mg/ml | 1 : 200 |
| Prolactin | 5µg/ml, c = 1mg/ml | 1 : 200 |
| Myostatin (GDF8) | 1µg/ml, c= 100µg/ml | 1 : 100 |

30 µl/well of the capture antibody dilution was added into the corresponding microplate wells. The wells where sealed with storage foil and spun down by centrifugation. The incubation was overnight at 4°C. Capture-antibody coated plates can be stored at 4°C for at least 4 weeks.

### (II) Sample Preparation and Incubation

All samples and standards were performed in duplicate. The standards were prepared fresh in appropriate standard-curve range for every plate. The standards are prepared in low-binding 1.5 ml vials. Below lists the amount of standard and buffer solution.

**Table 3: sample preparation**

| | |
|---|---|
| Mouse IgG | 1µl + 499µl 1 + 99 |
| Leptin | 8µl + 72µl |
| IGF 1 | 2.6µl + 77.3µl |
| Adiponectin | 1µl + 99µl 8 + 72 |
| Activin II B | 20µl + 180µl |
| Myostatin Propeptide | 20µl + 180µl |
| Prolactin | 20µl + 180µl |
| GDF8 | 7µl + 133µl |

The capture-antibody coated modules were washed with wash the program "BLOCK" (see below) in Imperacer® Washer. The washing steps were carried out using the Imperacer® Microplate Washer supplied the your Imperacer® Workstation (Cat. No. 25-001 or 25-002, Chimera Biotec, Germany).

240 µl/well of the Chimera Direct Block was added, and agitated for 30-60 seconds at room temperature with orbital shaking. After that, the wells were washed using the wash program "WASH" in Imperacer® Washer. 30µl of the diluted standards and samples were added to each well. The samples were incubated for 1 hour at room temperature and orbital shaking.

### (III) Imperacer® Conjugate Binding

The modules were washed after incubation using the wash program "Wash" in Imperacer® Washer. The appropriate Imperacer Conjugates were diluted in Conjugate Dilution Buffer "CDB" immediately prior to use, according to the dilution factors below.

**Table 4: Imperacer conjugates**

| **Antigen** | **Imperacer Conjugate** | **Dilution Factor** |
|---|---|---|
| Mouse IgG | CHI Mouse | 1 : 100 |
| Leptin | CHI Leptin | 1 : 1000 |
| IGF 1 | CHI IGF 1 | 1 : 30 |
| Adiponectin | CHI Adiponectin | 1 : 300 |
| Activin II B | Act II B | 1 : 30 |
| Myostatin Propeptide | CHI Myostatin | 1 : 300 |
| Prolactin | CHI Prolactin | 1 : 300 |
| GDF8 | CHI Mouse | 1 : 30 |

The conjugates were incubated with 30 µl/well of the diluted Imperacer® Conjugate solutions for 45 min at room temperature / orbital shaking.

### (IV) Signal read-out by real-time PCR

The wells in the modules were washed with the wash program "PCR" in the Imperacer® Washer. Modules were removed from the microplate module frame and placed in a 0.2 ml 96-well rack. 30 µl of the PCR-Mastermix were added into each well and the wells were sealed and subsequently transferred to the Imperacer® PCR instrument, using an Optical Cover Compression Pad on the sealed modules. Subsequently, the PCR program was started.

PCR was performed using an automated program, selecting FAM (emission at 518 nm) as fluorophore, and at 50°C in the PCR-cycle, using the following steps and repeats:

**Table 5: PCR conditions**

| *Time* | *Temperature* | *Repeats* |
|---|---|---|
| 4 min | 95°C | 1x |
| 12 sec | 95°C | 50x |
| 30 sec | 50°C | |
| 30 sec | 72°C | |

### 2.4 Results

**Table 6 of biomarker analysis**

| Sample | IgG anti-myostatin | Myostatin propetide | ActIIB receptor | Leptin |
|---|---|---|---|---|
| 2.1 (negative control) | o | o | o | o |
| 2.2 (positive control) | + | - | + | + |
| 2.3 (unknown) | + | - | + | + |
| 2.4 (unknown) | o | o | + | + |
| 2.5 (unknown) | o | + | + | - |

o = no change relative to negative control; + increase relative to negative control; - decrease relative to negative control

Table 6 shows the changes in amounts of the additional myostatin pathway biomarkers relative to a non-manipulated reference animal. The profile of the non-manipulated reference animal is characterized by a relatively high amount of myostatin propeptide, and relatively low amounts of actavin IIB receptor and leptin.

Applying a threshold of three standard deviations with respect to the reference animal provided a reliable means of analysing the data. Sample 2.1 was the negative reference (non-manipulated reference animal) and sample 2.2 was the positive control. Sample 2.3 has an increased amount of IgG anti-myostatin antibodies, Leptin and actavin IIB receptor compared to the reference sample, while the amount of myostatin propeptide is decreased.

Sample 2.4 shows an increased amount of anti-myostatin antibodies but the amount of myostatin propeptide is the same as in the non-manipulated reference animal. In this case the animal would be classed as suspicious and required to undergo further testing to conclusively classify this an animal which has been subjected to muscle growth manipulation.

Sample 2.5 shows that an animal wherein the amount of IgG anti-myostatin antibodies remains the same as the reference, the amounts of the additional biomarkers do not follow the same pattern as when an increased amount of IgG anti-myostatin antibodies is present.

Example 2 therefore shows that by determining the changes in the amounts of additional myostatin pathway proteins relative to the amounts of these biomarkers in reference animals, the presence of muscle growth manipulation can be determined.

Example 2 also shows that the relationship between the amount of anti-myostatin antibodies and the additional myostatin pathway biomarkers is also indicative for muscle growth manipulation via the myostatin pathway. In other words, there is a unique fingerprint for the manipulation via the myostatin pathway, whereby when the amount of anti-myostatin antibodies increases, there is also an increase in activin IIB receptor and leptin, while myostatin propeptide decreases.

By correlating the amount of additional biomarkers to the amount of anti-myostatin anti-bodies, the presence of muscle growth manipulation can be distinguished from pathological processes, such as in samples 2.4 and 2.5 where there is a different profile of additional myostatin biomarkers but no change in the amount of anti-myostatin antibodies.

In this way the method is able to distinguish between animals that have been subjected to muscle growth manipulation via the myostatin pathway, for example sample 2.3, and animals that have different amounts of additional myostatin pathway biomarkers as a result of pathological processes, such as sample 2.4.

### Example 3: Quantification of additional myostatin pathway related biomarkers in calves

Example 2 was repeated using bovine samples analysed in Example 1.

Detection Conjugate "CHI-Bovine-IgG" (conjugated antibody: anti-Bovine IgG-heavy and light chain Antibody, Sheep Polyclonal, catalogue number: A10-115; Supplier: Bethyl Laboratories, Inc., USA) was used instead of the mouse conjugate. All other Imperacer conjugates for the additional myostatin pathway biomarkers were prepared using standard methods as described in EP 2189539 ([0106]-[0122]). For the biomarkers p-AKT, p-FOXO, MyOD1, MyoG, FBO32, ALK type 1, Pax3, Myf-5, MuRF1, CDKN1 and CDK2, tissue homogenate instead of serum was used.

**Table 7: Biomarker analysis**

| Sample | IgG anti-myostatin antibodies | (P)-SMAD2/ SMAD 3 | Leptin | Act IIB | Myostatin Propeptide | p-AKT | (p)-FOxO | MyoD1 | MyoG | FBXO32 | ALK type 1 | Pax 3 | Myf-5 | MuRF1 | CDKN1 A | CDK2 |
|---|---|---|---|---|---|---|---|---|---|---|---|---|---|---|---|---|
| 1.1 | o | o | o | o | o | o | o | o | o | o | o | o | o | o | o | o |
| 1.2 | + | - | + | + | - | + | + | + | + | - | - | + | + | - | + | + |
| 1.3 | + | - | + | + | - | + | + | + | + | - | - | + | + | - | + | + |
| 1.4 | + | MyoD1 | + | + | MyoD1 | + | + | + | + | - | - | + | + | - | + | + |
| 1.5 | o | + | o | + | + | - | - | o | o | o | o | o | - | - | - | + |

| | | | | | | | | | | | | | | | | |
|---|---|---|---|---|---|---|---|---|---|---|---|---|---|---|---|---|
| (p) = phosphorylated + increase, - decrease, ○ no change | | | | | | | | | | | | | | | | |

The additional myostatin pathway biomarkers shown in Table 7 were quantified using immune-PCR. By measuring the amount of these biomarkers of the myostatin pathway it is possible to determine whether a suspicious sample (e.g. sample 1.4) has in fact been subjected to muscle growth manipulation. Table 7 shows the change in the biomarkers measured relative to the non-manipulated reference animal.

The additional myostatin pathway biomarker profile for a sample taken from a non-manipulated reference animal is characterized by relatively high amounts of phosphorylated SMAD2 and SMAD3, myostatin propeptide, FBXO32, ALK type 1 co-receptor and MuRF1, together with relatively low amounts of leptin, ActIIB, p-AKT, MyoD1, MyoG, PAx3, Myf-5 and phosphorylated-FOXO.

As can be seen in table 7, the amount of phosphorylated SMAD2, FBXO32 and myostatin propeptide, ALK, MuRF1 decrease in the sample 1.3 and 1.4 relative to the non-manipulated reference mirroring the decrease in the positive control sample 1.2. Phosphorylated SMAD2, FBXO32, myostatin propeptide, ALK, MuRF1 are therefore examples of additional biomarkers of the myostatin pathway that can be used to identify the presence of the manipulation of muscle mass by manipulation of the myostatin pathway.

With respect to biomarkers leptin, ActIIB, p-AKT, MyoD1, MyoG, PAx3, Myf-5, P-FOXO, an increased amount of these biomarkers is observed in samples 1.3 and 1.4 relative to the reference, but no change is observed in sample 1.5.

By comparing the amount of the additional biomarkers of the myostatin pathway in a sample to those of a reference sample, it is possible to determine whether the sample has also been subjected to artificial muscle growth manipulation. For example, sample 1.3 was determined positive in Example 1, and that is confirmed in this example.

Example 3 shows that correlating the amount of anti-myostatin antibodies to the amount of additional biomarkers can determine whether an animal has been subject to muscle growth manipulation or not, as shown by samples 1.4 and 1.5.

A further means of confirming the sample status is the relationship between the respective additional myostatin pathway biomarkers. Sample 1.4, which was determined "suspicious" in Example 1, can be classified as "positive" because this sample not only has an increase in the amount of anti-myostatin antibodies but also the pattern of changes in biomarkers differs from that of the pattern in the non-manipulated reference animal. This finding is confirmed by the observation that sample 1.4 has the same additional myostatin pathway biomarker profile as for the reference sample 1.2 (positive control). The means that the relationship of the respective additional myostatin pathway biomarkers is helpful in determining whether there is has been muscle growth manipulation.

The results as shown in Table 7 show that the profile generated by analysing additional myostatin pathway biomarkers can be used to identify whether a domestic animal has been subjected to muscle growth manipulation or not. A profile indicative of muscle growth manipulation when compared to a non-manipulated reference animal, in this case sample 1.1, is characterised by a decrease in the amount of phosphorylated SMAD2 and SMAD3, myostatin propeptide, FBXO32, ALK type 1 receptor and MurF1. The decrease in these biomarkers is accompanied by an increase in the amount of leptin, ActIIB, p-AKT, phosphorylated FOXO, MyoD1, MyoG, Pax3, Myf-5, CDKN1 and CDK2. This profile is present in 1.3 and 1.4 but absent in sample 1.5 thus the method according to the present invention can distinguish between animals that have been subjected to muscle growth manipulation and those that have not been subjected to muscle growth manipulation.

In summary, Example 3 shows that it is possible to determine the presence of muscle growth manipulation by determining the following relationships, all relative to a non-manipulated reference:
i) the amount of anti-myostatin antibodies;
ii) the amount of at least one additional myostatin pathway biomarker and the profile generated by determining the increase or decrease in the amounts of these biomarkers relative to a non-manipulated reference;
iii) the correlation between relationship i) and ii);

## Claims

1. Diagnostic method for the detection of the manipulation of muscle growth in a domestic animal, comprising the steps of:
a) providing a tissue or body fluid sample obtained from a domestic animal,
b) quantifying the amount of anti-myostatin antibodies in the sample of step a),
c) comparing the amount of the anti-myostatin antibodies measured in step b) to the amount of anti-myostatin antibodies present in at least one non-manipulated reference animal, wherein an increased amount of the anti-myostatin antibodies present in the sample relative to the at least one non-manipulated reference animal is indicative for the manipulation of muscle growth in a domestic animal,
wherein the method further comprises the steps of:
d) quantifying the amount of at least one additional myostatin pathway biomarker in the said sample, the said additional myostatin pathway biomarker being selected from the group consisting of myostatin propeptide, leptin, activin receptor IIB, members of the SMAD family of proteins, Alk type 1 co-receptor, Pax 3, MYOD1, MYOG, CDKN1A, CDK2 Myf-5, p-AKT, FOXO, MuRF1, FBXO32 and combinations of two or more thereof,
e) comparing the amount of the at least one additional myostatin pathway biomarker measured in step d) to the amount of the said at least one additional myostatin pathway biomarker present in at least one non-manipulated reference animal, wherein an altered amount of the said additional myostatin pathway biomarker present in the sample relative to the at least one non-manipulated reference animal is indicative for the manipulation of muscle growth in a domestic animal.

2. Diagnostic method according to claim 1, wherein the anti-myostatin antibodies are chosen from the group consisting of IgG, IgA, IgD, IgE or IgM and IgG anti-myostatin antibodies, preferably an IgG anti-myostatin antibody.

3. Diagnostic method according to claim 1, wherein step d) comprises at least two, preferably at least three myostatin pathway biomarkers and wherein step e) comprises comparing the amount of the at least two myostatin pathway biomarkers measured in step d) to the amount of the said at least two, preferably the at least three additional myostatin pathway biomarkers present in at least one non-manipulated reference animal, respectively.

4. Diagnostic method according to claim 1 or 3, wherein the method further comprises the steps of:
f) correlating the amount of anti-myostatin antibodies quantified in step b) with the amount of one or more, preferably at least two additional myostatin pathway biomarkers quantified in step d) therewith providing a profile of the relationship between the relative amounts of the said anti-myostatin antibodies and the said one or more additional myostatin pathway biomarkers,
g) comparing the profile in step f) to a reference profile obtained by correlating the amount of said anti-myostatin antibodies with the amount said of one or more additional myostatin pathway biomarkers quantified in at least one non-manipulated reference animal,
wherein the manipulation of muscle growth in a domestic animal can be determined when the profile of step f) differs from that of step g).

5. Diagnostic method according to any of the preceding claims, wherein the domestic animal is:
- an animal raised for meat production, and/or
- an animal selected from the group consisting of cattle, sheep, horse, goat, pigs, poultry, lama, ostrich and fish, preferably cattle, and/or
- a young animal, preferably younger than one year old.

6. Diagnostic method according to any of the preceding claims, wherein the reference animal is of the same species as the domestic animal, preferably sub species, more preferably the same breed, yet more preferably also the same gender and even more preferably also the same age.

7. Diagnostic method according to any of the preceding claims, wherein the sample is:
- a bodily fluid or a soft tissue, the bodily fluid preferably being selected from the group consisting of saliva, sweat, urine, whole blood, blood serum, blood plasma and cerebrospinal fluid, the sample preferably comprising blood serum, the soft tissue preferably being muscle tissue.

8. Diagnostic method according to any one of the preceding claims, wherein step b) comprises the steps of:
i) providing a solid surface, preferably a micro-titre plate, comprising a first binding agent capable of specifically binding the anti-myostatin antibodies to be quantified,
ii) contacting the solid surface with the sample from the domestic animal,
iii) washing the surface obtained in step ii),
iv) contacting the surface obtained in step iii) with a first detector agent capable of specifically binding the anti-myostatin antibodies,
v) quantifying the signal emitted by the first detector agent.

9. Diagnostic method according claim 8, wherein the first binding agent is an antibody, epitope or protein fragment capable of specifically binding the anti-myostatin antibodies, the first binding agent preferably comprising a crosslinking reagent for crosslinking with the first detector agent in the presence of the anti-myostatin antibodies, the crosslinking agent preferably being biotin.

10. Diagnostic method according to claim 8, the surface further comprises at least one additional binding agent capable of specifically binding the at least one additional myostatin pathway biomarker, the at least one additional binding agent preferably being an antibody, epitope or protein fragment capable of specifically binding the at least one additional myostatin pathway biomarker, step iv) preferably comprising contacting the sample with at least one additional detector agent capable of specifically binding the at least one additional myostatin pathway biomarker.

11. Diagnostic method according to any one of claims 8 to 10, wherein the first detector agent, and optionally the at least one additional detector agent, comprises a means to generate a quantifiable signal, said means being selected from the group consisting of polynucleotides detectable by immuno-PCR, fluorophores detectable via fluorescence spectroscopy and enzymes detectable via UV spectroscopy, said means to generate a quantifiable signal preferably being provided by polynucleotides.

12. Diagnostic kit for performing a diagnostic method according to any of the preceding claims, wherein the kit comprises:
i) a solid surface comprising at least a first binding agent capable of specifically binding anti-myostatin antibodies,
ii) a holder comprising a first detector agent capable of specifically binding anti-myostatin antibodies, and optionally
iii) a set of instructions for the use of the kit containing values for the minimum amount of anti-myostatin antibodies that are required to be present in the sample for manipulation of muscle growth to be determined,
wherein the kit further comprises:
- at least one additional binding agent capable of specifically binding at least one additional myostatin pathway biomarker to be quantified and at least one additional detector agent capable of specifically binding at least one additional myostatin pathway biomarker to be quantified.

13. Diagnostic kit according to claim 13, wherein the kit further comprises:
- reference data for the amount of the at least one additional myostatin pathway biomarker and optionally further comprises one or more reference profiles of non-manipulated reference animals, said reference data and/or reference profiles being provided in the set of instructions or on a computer readable medium supplied with the kit.

14. Diagnostic kit according to claim 12 or 13, wherein the first detector agent and optionally the at least one additional detector agent comprises a means to generate a quantifiable signal, said means being selected from the group consisting of polynucleotides detectable by immuno-PCR, fluorophores detectable via fluorescence spectroscopy and enzymes detectable via UV spectroscopy.

## Patentansprüche

1. Diagnoseverfahren für den Nachweis der Manipulation des Muskelwachstums bei einem Haustier, umfassend die folgenden Schritte:
a) Bereitstellen einer Gewebe- oder Körperflüssigkeitsprobe, die von einem Haustier erhalten wurde,
b) Quantifizieren der Menge an Anti-Myostatin-Antikörpern in der Probe von Schritt a),
c) Vergleichen der Menge der in Schritt b) gemessenen Anti-Myostatin-Antikörper mit der Menge der in mindestens einem nicht-manipulierten Referenztier vorhandenen Anti-Myostatin-Antikörper, wobei eine erhöhte Menge der in der Probe vorhandenen Anti-Myostatin-Antikörper relativ zu dem mindestens einen nicht-manipulierten Referenztier ein Hinweis auf die Manipulation des Muskelwachstums bei einem Haustier ist, wobei das Verfahren ferner die folgenden Schritte umfasst:
d) Quantifizieren der Menge von mindestens einem zusätzlichen Myostatinweg-Biomarker in der Probe, wobei der zusätzliche Myostatinweg-Biomarker ausgewählt ist aus der Gruppe bestehend aus Myostatin-Propeptid, Leptin, Aktivinrezeptor IIB, Mitgliedern der SMAD-Proteinfamilie, Alk-Typ-1-Corezeptor, Pax 3, MYOD1, MYOG, CDKN1A, CDK2 Myf-5, p-AKT, FOXO, MuRF1, FBXO32 und Kombinationen von zwei oder mehr davon,
e) Vergleichen der Menge des mindestens einen zusätzlichen Myostatinweg-Biomarkers, gemessen in Schritt d), mit der Menge des mindestens einen zusätzlichen Myostatinweg-Biomarkers, der in mindestens einem nicht-manipulierten Referenztier vorhanden ist, wobei eine veränderte Menge des in der Probe vorhandenen genannten einen zusätzlichen Myostatinweg-Biomarkers relativ zu dem mindestens einen nicht-manipulierten Referenztier ein Hinweis auf die Manipulation des Muskelwachstums bei einem Haustier ist.

2. Diagnoseverfahren nach Anspruch 1, wobei die Anti-Myostatin-Antikörper ausgewählt sind aus der Gruppe bestehend aus IgG-, IgA-, IgD-, IgE- oder IgM- und IgG-Anti-Myostatin-Antikörpern, vorzugsweise einem IgG-Anti-Myostatin-Antikörper.

3. Diagnoseverfahren nach Anspruch 1, wobei Schritt d) mindestens zwei, vorzugsweise mindestens drei Myostatinweg-Biomarker umfasst und wobei Schritt e) das Vergleichen der Menge der mindestens zwei Myostatinweg-Biomarker, gemessen in Schritt d), mit der Menge von den genannten mindestens zwei, vorzugsweise mindestens drei zusätzlichen Myostatinweg-Biomarkern, die jeweilig in mindestens einem nicht-manipulierten Referenztier vorhanden sind, umfasst.

4. Diagnoseverfahren nach Anspruch 1 oder 3, wobei das Verfahren ferner die Schritte umfasst:
f) Korrelieren der Menge an Anti-Myostatin-Antikörpern, die in Schritt b) quantifiziert wurden, mit der Menge von einem oder mehreren, vorzugsweise mindestens zwei zusätzlichen Myostatinweg-Biomarkern, die in Schritt d) quantifiziert wurden, um dadurch ein Profil der Beziehung zwischen den relativen Mengen der genannten Anti-Myostatin-Antikörper und der genannten einen oder mehreren zusätzlichen Myostatinweg-Biomarker zu erhalten,
g) Vergleichen des Profils in Schritt f) mit einem Referenzprofil, das durch Korrelieren der Menge der genannten Anti-Myostatin-Antikörper mit der Menge der genannten ein oder mehreren zusätzlichen Myostatinweg-Biomarker erhalten wurde, die in mindestens einem nicht-manipulierten Referenztier quantifiziert wurden,
wobei die Manipulation des Muskelwachstums bei einem Haustier festgestellt werden kann, wenn sich das Profil von Schritt f) von demjenigen von Schritt g) unterscheidet.

5. Diagnoseverfahren nach einem der vorhergehenden Ansprüche, wobei es sich bei dem Haustier handelt um:
- ein zur Fleischproduktion aufgezogenes Tier und/oder
- ein Tier, ausgewählt aus der Gruppe bestehend aus Rindern, Schafen, Pferden, Ziegen, Schweinen, Geflügel, Lamas, Straußen und Fischen, vorzugsweise Rindern, und/oder
- ein junges Tier, das vorzugsweise jünger als ein Jahr alt ist.

6. Diagnoseverfahren nach einem der vorhergehenden Ansprüche, wobei das Referenztier von derselben Spezies, vorzugsweise Subspezies, bevorzugter derselben Rasse, noch bevorzugter auch demselben Geschlecht und noch stärker bevorzugt auch demselben Alter wie das Haustier ist.

7. Diagnoseverfahren nach einem der vorhergehenden Ansprüche, wobei es sich bei der Probe handelt um:
- eine Körperflüssigkeit oder ein Weichgewebe, wobei die Körperflüssigkeit vorzugsweise ausgewählt ist aus der Gruppe bestehend aus Speichel, Schweiß, Urin, Vollblut, Blutserum, Blutplasma und Liquor cerebrospinalis, wobei die Probe vorzugsweise Blutserum umfasst, wobei das Weichgewebe vorzugsweise Muskelgewebe ist.

8. Diagnoseverfahren nach einem der vorhergehenden Ansprüche, wobei Schritt b) die folgenden Schritte umfasst:
i) Bereitstellen einer festen Oberfläche, vorzugsweise einer Mikrotiterplatte, umfassend ein erstes Bindemittel, das in der Lage ist, die zu quantifizierenden Anti-Myostatin-Antikörper spezifisch zu binden,
ii) Inkontaktbringen der festen Oberfläche mit der Probe aus dem Haustier,
iii) Waschen der in Schritt ii) erhaltenen Oberfläche,
iv) Inkontaktbringen der in Schritt iii) erhaltenen Oberfläche mit einem ersten Detektormittel, das in der Lage ist, die Anti-Myostatin-Antikörper spezifisch zu binden,
v) Quantifizieren des vom ersten Detektormittel emittierten Signals.

9. Diagnoseverfahren nach Anspruch 8, wobei das erste Bindemittel ein Antikörper, ein Epitop oder ein Proteinfragment ist, das in der Lage ist, die Anti-Myostatin-Antikörper spezifisch zu binden, wobei das erste Bindemittel vorzugsweise ein Vernetzungsreagenz zur Vernetzung mit dem ersten Detektormittel in Gegenwart der Anti-Myostatin-Antikörper umfasst, wobei das Vernetzungsmittel vorzugsweise Biotin ist.

10. Diagnoseverfahren nach Anspruch 8, wobei die Oberfläche ferner mindestens ein zusätzliches Bindemittel umfasst, das in der Lage ist, den mindestens einen zusätzlichen Myostatinweg-Biomarker spezifisch zu binden, wobei das mindestens eine zusätzliche Bindemittel vorzugsweise ein Antikörper, Epitop oder Proteinfragment ist, das dazu in der Lage ist, den mindestens einen zusätzlichen Myostatinweg-Biomarker spezifisch zu binden, wobei der Schritt iv) vorzugsweise das Inkontaktbringen der Probe mit mindestens einem zusätzlichen Detektormittel umfasst, das in der Lage ist, den mindestens einen zusätzlichen Myostatinweg-Biomarker spezifisch zu binden.

11. Diagnoseverfahren nach einem der Ansprüche 8 bis 10, wobei das erste Detektormittel und gegebenenfalls das mindestens eine zusätzliche Detektormittel ein Hilfsmittel zum Erzeugen eines quantifizierbaren Signals umfasst, wobei das genannte Hilfsmittel ausgewählt ist aus der Gruppe, bestehend aus Polynukleotiden, die nachweisbar sind durch Immun-PCR, Fluorophore, die nachweisbar sind durch Fluoreszenzspektroskopie, und Enzymen, die nachweisbar sind durch UV-Spektroskopie, wobei das genannte Hilfsmittel zur Erzeugung eines quantifizierbaren Signals vorzugsweise durch Polynukleotide bereitgestellt wird.

12. Diagnosekit zum Durchführen eines Diagnoseverfahrens gemäß einem der vorhergehenden Ansprüche, wobei das Kit umfasst:
i) eine feste Oberfläche, umfassend mindestens ein erstes Bindemittel, das in der Lage ist, die Anti-Myostatin-Antikörper spezifisch zu binden,
ii) einen Halter, umfassend ein erstes Detektormittel, das in der Lage ist, Anti-Myostatin-Antikörper spezifisch zu binden, und gegebenenfalls
iii) einen Satz von Anweisungen für die Verwendung des Kits, enthaltend Werte für die Mindestmenge an Anti-Myostatin-Antikörpern, die zum Feststellen einer Manipulation des Muskelwachstums in der Probe vorhanden sein muss, wobei das Kit ferner umfasst:
- mindestens ein zusätzliches Bindemittel, das in der Lage ist, mindestens einen zusätzlichen zu quantifizierenden Myostatinweg-Biomarker spezifisch zu binden, und mindestens ein zusätzliches Detektormittel, das in der Lage ist, mindestens einen zusätzlichen zu quantifizierenden Myostatinweg-Biomarker spezifisch zu binden.

13. Diagnosekit nach Anspruch 13, wobei das Kit ferner umfasst:
- Referenzdaten für die Menge des mindestens einen zusätzlichen Myostatinweg-Biomarkers, und gegebenenfalls ferner umfassend ein oder mehrere Referenzprofile von nicht-manipulierten Referenztieren, wobei die genannten Referenzdaten und/oder Referenzprofile in dem Satz von Anweisungen oder auf einem Computer-lesbaren Medium, welche(s) mit dem Kit mitgeliefert wurde(n), bereitgestellt werden.

14. Diagnosekit nach Anspruch 12 oder 13, wobei das erste Detektormittel und gegebenenfalls das mindestens eine zusätzliche Detektormittel ein Hilfsmittel zum Erzeugen eines quantifizierbaren Signals umfasst, wobei das genannte Hilfsmittel ausgewählt ist aus der Gruppe bestehend aus Polynukleotiden, die nachweisbar sind durch Immun-PCR, Fluorophore, die nachweisbar sind durch Fluoreszenzspektroskopie, und Enzymen, die nachweisbar sind durch UV-Spektroskopie.

## Revendications

1. Procédé de diagnostic pour la détection de la manipulation de la croissance musculaire chez un animal domestique, comprenant les étapes consistant :
a) à fournir un échantillon de fluide corporel ou de tissu obtenu à partir d'un animal domestique,
b) à quantifier la quantité d'anticorps anti-myostatine dans l'échantillon de l'étape a),
c) à comparer la quantité des anticorps anti-myostatine mesurée dans l'étape b) à la quantité d'anticorps anti-myostatine présents chez au moins un animal de référence non manipulé, où une quantité accrue des anticorps anti-myostatine présents dans l'échantillon par rapport à l'au moins un animal de référence non manipulé indique la manipulation de la croissance musculaire chez un animal domestique,
dans lequel le procédé comprend en outre les étapes consistant :
d) à quantifier la quantité d'au moins un biomarqueur de la voie de myostatine supplémentaire dans ledit échantillon, ledit biomarqueur de la voie de myostatine supplémentaire étant choisi dans le groupe constitué par le propeptide de myostatine, la leptine, le récepteur de l'activine IIB, des membres de la famille de protéines SMAD, le co-récepteur Alk de type 1, Pax 3, MYOD1, MYOG, CDKN1A, CDK2 Myf-5, p-AKT, FOXO, MuRF1, FBXO32 et des combinaisons de deux de ceux-ci ou plus,
e) à comparer la quantité de l'au moins un biomarqueur de la voie de myostatine supplémentaire mesurée dans l'étape d) à la quantité dudit au moins un biomarqueur de la voie de myostatine supplémentaire présent chez au moins un animal de référence non manipulé, où une quantité modifiée dudit biomarqueur de la voie de myostatine supplémentaire présent dans l'échantillon par rapport à l'au moins un animal de référence non manipulé indique la manipulation de la croissance musculaire chez un animal domestique.

2. Procédé de diagnostic selon la revendication 1, dans lequel les anticorps anti-myostatine sont choisis dans le groupe constitué par IgG, IgA, IgD, IgE ou IgM et des anticorps anti-myostatine IgG, de préférence un anticorps anti-myostatine IgG.

3. Procédé de diagnostic selon la revendication 1, dans lequel l'étape d) comprend au moins deux, de préférence au moins trois biomarqueurs de la voie de myostatine et dans lequel l'étape e) comprend le fait de comparer la quantité des au moins deux biomarqueurs de la voie de myostatine mesurée dans l'étape d) à la quantité desdits au moins deux, de préférence des au moins trois biomarqueurs de la voie de myostatine supplémentaires présents chez au moins un animal de référence non manipulé, respectivement.

4. Procédé de diagnostic selon la revendication 1 ou 3, dans lequel le procédé comprend en outre les étapes consistant :
f) à mettre en corrélation la quantité d'anticorps anti-myostatine quantifiée dans l'étape b) avec la quantité d'un ou de plusieurs, de préférence d'au moins deux biomarqueurs de la voie de myostatine supplémentaires quantifiée dans l'étape d) fournissant ainsi un profil de la relation entre les quantités relatives desdits anticorps anti-myostatine et dudit ou desdits plusieurs biomarqueur(s) de la voie de myostatine supplémentaire(s),
g) à comparer le profil dans l'étape f) à un profil de référence obtenu par corrélation de la quantité desdits anticorps anti-myostatine avec la quantité dudit ou desdits plusieurs biomarqueur(s) de la voie de myostatine supplémentaire(s) quantifiée chez au moins un animal de référence non manipulé,
dans lequel la manipulation de la croissance musculaire chez un animal domestique peut être déterminée lorsque le profil de l'étape f) diffère de celui de l'étape g).

5. Procédé de diagnostic selon l'une des revendications précédentes, dans lequel l'animal domestique est :
- un animal élevé pour la production de viande, et/ou
- un animal choisi dans le groupe constitué par un bovin, un mouton, un cheval, une chèvre, des porcs, une volaille, un lama, une autruche et un poisson, de préférence un bovin, et/ou
- un jeune animal, de préférence âgé de moins d'un an.

6. Procédé de diagnostic selon l'une des revendications précédentes, dans lequel l'animal de référence est de la même espèce que l'animal domestique, de préférence de la même sous-espèce, plus préférablement de la même race, encore plus préférablement aussi du même sexe et mieux encore aussi du même âge.

7. Procédé de diagnostic selon l'une des revendications précédentes, dans lequel l'échantillon est :
- un fluide corporel ou un tissu mou, le fluide corporel étant de préférence choisi dans le groupe constitué par la salive, la sueur, l'urine, le sang total, le sérum sanguin, le plasma sanguin et le liquide céphalorachidien, l'échantillon comprenant de préférence du sérum sanguin, le tissu mou étant de préférence un tissu musculaire.

8. Procédé de diagnostic selon l'une quelconque des revendications précédentes, dans lequel l'étape b) comprend les étapes consistant :
i) à fournir une surface solide, de préférence une plaque de microtitration, comprenant un premier agent de liaison capable de se lier spécifiquement aux anticorps anti-myostatine à quantifier,
ii) à mettre en contact la surface solide avec l'échantillon provenant de l'animal domestique,
iii) à laver la surface obtenue dans l'étape ii),
iv) à mettre en contact la surface obtenue dans l'étape iii) avec un premier agent de détection capable de se lier spécifiquement aux anticorps anti-myostatine,
v) à quantifier le signal émis par le premier agent de détection.

9. Procédé de diagnostic selon la revendication 8, dans lequel le premier agent de liaison est un anticorps, un épitope ou un fragment de protéine capable de se lier spécifiquement aux anticorps anti-myostatine, le premier agent de liaison comprenant de préférence un réactif de réticulation pour se réticuler avec le premier agent de détection en présence des anticorps anti-myostatine, l'agent de réticulation étant de préférence la biotine.

10. Procédé de diagnostic selon la revendication 8, la surface comprend en outre au moins un agent de liaison supplémentaire capable de se lier spécifiquement à l'au moins un biomarqueur de la voie de myostatine supplémentaire, l'au moins un agent de liaison supplémentaire étant de préférence un anticorps, un épitope ou un fragment de protéine capable de se lier spécifiquement à l'au moins un biomarqueur de la voie de myostatine supplémentaire, l'étape iv) comprenant de préférence le fait de mettre en contact l'échantillon avec au moins un agent de détection supplémentaire capable de se lier spécifiquement à l'au moins un biomarqueur de la voie de myostatine supplémentaire.

11. Procédé de diagnostic selon l'une quelconque des revendications 8 à 10, dans lequel le premier agent de détection, et facultativement l'au moins un agent de détection supplémentaire, comprend un moyen de génération d'un signal quantifiable, ledit moyen étant choisi dans le groupe constitué par des polynucléotides détectables par immuno-PCR, des fluorophores détectables par spectroscopie de fluorescence et des enzymes détectables par spectroscopie UV, ledit moyen de génération d'un signal quantifiable étant de préférence fourni par des polynucléotides.

12. Kit de diagnostic pour réaliser un procédé de diagnostic selon l'une des revendications précédentes, dans lequel le kit comprend :
i) une surface solide comprenant au moins un premier agent de liaison capable de se lier spécifiquement aux anticorps anti-myostatine,
ii) un support comprenant un premier agent de détection capable de se lier spécifiquement aux anticorps anti-myostatine, et facultativement
iii) un ensemble d'instructions pour l'utilisation du kit contenant des valeurs pour la quantité minimale d'anticorps anti-myostatine qui doivent être présents dans l'échantillon pour la manipulation de la croissance musculaire à déterminer,
dans lequel le kit comprend en outre :
- au moins un agent de liaison supplémentaire capable de se lier spécifiquement à au moins un biomarqueur de la voie de myostatine supplémentaire à quantifier et au moins un agent de détection supplémentaire capable de se lier spécifiquement à au moins un biomarqueur de la voie de myostatine supplémentaire à quantifier.

13. Kit de diagnostic selon la revendication 13, dans lequel le kit comprend en outre :
- des données de référence pour la quantité de l'au moins un biomarqueur de la voie de myostatine supplémentaire et comprend en outre facultativement un ou plusieurs profil(s) de référence d'animaux de référence non manipulés, lesdites données de référence et/ou lesdits profils de référence étant fourni(e)s dans l'ensemble d'instructions ou sur un support lisible par ordinateur fourni avec le kit.

14. Kit de diagnostic selon la revendication 12 ou 13, dans lequel le premier agent de détection, et facultativement l'au moins un agent de détection supplémentaire, comprend un moyen de génération d'un signal quantifiable, ledit moyen étant choisi dans le groupe constitué par des polynucléotides détectables par immuno-PCR, des fluorophores détectables par spectroscopie de fluorescence et des enzymes détectables par spectroscopie UV.


### SEQUENCE LISTING

<110> Stichting Kwaliteitsgarantie Vleeskalversector
<120> Diagnostic method for the detection of the manipulation of muscle
   growth in a domestic animal
<130> 61030PC
<150> EP14173757.7
   <151> 2014-06-24
<160> 1
<170> PatentIn version 3.5
<210> 1
   <211> 20
   <212> PRT
   <213> Artificial Sequence
<220>
   <223> Synthetic peptide
<400> 1
   Cys Lys Met Ser Pro Ile Asn Met Leu Tyr Phe Asn Gly Lys Glu Gln
   1               5                   10                  15
   Ile Ile Tyr Gly
               20